# EUROPEAN PATENT SPECIFICATION

(11) **EP 1 904 149 B1**
(45) Date of publication and mention of the grant of the patent: **29.09.2010**
(21) Application number: 06761223.4
(22) Date of filing: 17.07.2006
(51) Int. Cl.: A61M 35/00, A61B 18/02

(54) **SPRAY DEVICE FOR DISPENSING A COOLING FLUID**
SPRÜHVORRICHTUNG ZUR ABGABE EINER KÜHLENDEN FLÜSSIGKEIT
DISPOSITIF DE PULVERISATION SERVANT A DISTRIBUER UN FLUIDE REFRIGERANT

(30) Priority: 24.08.2005 US 210339; 18.07.2005 CH 11942005
(43) Date of publication of application: 02.04.2008
(60) Divisional application: 10156673.5
(73) Proprietor: Aerosol-Service AG, CH-4313 Möhlin (CH)
(72) Inventor: GEIGER, Jörg, 79730 Murg-Niederhof (DE); PATRICK, Campbell, 23843 Bad Oldesloe (DE)
(74) Representative: Bohest AG
(86) International application number: PCT/CH2006/000372
(87) International publication number: WO 2007/009282

(56) References cited:
- GB-A- 1 163 573
- US-A- 5 516 505

## Description

The invention relates to a spray device for dispensing a cooling fluid in accordance with claim 1, and with a method of treating skin defects in accordance with claim 15.

Spray devices of this kind are already well known and are used in various fields. For example, they are used to remove skin defects, such as warts, as is described in EP-A-0 281 212 or in EP-A-0 608 954. In the spray devices described in these documents, a fluid outlet valve is actuated so that the cooling fluid stored in liquid form in a container is first transported through a tube from the container into the valve chamber and from there through a further tube to a cotton bud or a plug of open-cell foam mounted on the tube at the outlet end. The liquid cooling fluid, which has a boiling point of below 0°C, typically in the range of between -50°C and 0°C, soaks the cotton bud or passes into the plug of open-cell foam and evaporates, as a result of which the cotton bud or the plug of open-cell foam is supercooled. This cotton bud or plug of open-cell foam is then brought into contact with the wart to be treated, which procedure can be done very precisely according to the nature of the geometric configuration of the cotton bud or of the plug. However, these devices have the disadvantage that comparatively large amounts of cooling fluid are needed for a single treatment.

WO-A-03/051522 proposes a spray device in which a liquid to be dispensed is stored in a container and is then applied in the form of an aerosol with the aid of a propellant. However, propellants often pose a problem with regard to the environment. To reduce the required amount of propellant while maintaining the same amount of liquid to be dispensed, WO-A-03/051522 thus proposes providing the spray head with a capillary tube into which the liquid to be dispensed and the propellant are introduced either separately or together. In the capillary tube, the liquid and the propellant form an aerosol which is made up of fine droplets and which can then be dispensed. A correspondingly suitable spray head is described in EP-A-1 516 829.

A device and method for treating skin lesions are disclosed in US 5,516,505. For the treatment of the skin lesion a liquid cryogenic agent is dispensed through a capillary tube into a synthetic plastic foam applicator where it is allowed to evaporate. The thus cooled plastic foam applicator is pressed against the skin lesion.

A device for treating skin defects according to the preamble of claim 1 is disclosed. GB 1,163,573.

The object of the invention is to propose a spray device for a coolant, which spray device represents an improvement on the prior art and, in order to ensure very efficient cooling of a target, for example a skin defect (e.g. a wart), dispenses an as small as possible amount of coolant.

According to the invention, this is achieved by a spray device as characterized by the features of independent claim 1. Advantageous developments of the spray device according to the invention form the subject of the related dependent claims.

For this purpose, in particular, not only is there a capillary tube arranged in the spray head, there is also a further capillary tube (as riser tube) arranged in the reservoir. The inlet end of this capillary tube arranged in the reservoir extends into the cooling fluid stored in liquid form in the reservoir. The outlet end of the capillary tube is connected to the fluid outlet valve. Therefore, when the valve is actuated, an aerosol is already generated in the capillary tube, and this aerosol further evaporates and then passes through the fluid outlet valve (which has a very small valve chamber whose volume is at any rate less than 50 mm³) into the capillary tube in the spray head.

At the outlet end of the capillary tube of the spray head, the cooling fluid is ready in essentially gaseous form and emerges from the capillary tube of the spray head. In the expansion that then occurs, the cooling fluid cools down very sharply, so that very efficient cooling takes place and, at the same time, only a comparatively small amount of cooling fluid is needed for this efficient cooling.

The internal diameter of the capillary tubes is in the range of 0.35 mm to 1 mm. Particularly efficient cooling is in this way ensured, with at the same time a low consumption of cooling fluid.

It may be advantageous if the spray head is provided with a receiver opening for receiving an applicator in which the fluid is guided to an intended target. For example, in the treatment of skin defects (e.g. warts), it is thus desirable to direct the cooling fluid with the greatest possible accuracy toward the target in order to avoid involvement of healthy tissue as far as possible.

Such an applicator can have a pipe section which is firmly connected to the spray head, this being made possible, for example, by a snap-fit connection to the spray head. Such a connection is reliable and, if appropriate, can also be undone again. The pipe section can itself be designed in a particular way, or can be provided with further means, allowing the cooling fluid to be dispensed accurately toward the target, as will be explained below.

Thus, in an illustrative embodiment of the spray device according to the invention, the pipe section has, at its free end, a bell-shaped end area whose internal diameter is greater than the internal diameter of the remaining pipe section. The capillary tube of the spray head is guided through the pipe section, and its outlet end is arranged in the bell-shaped end area of the pipe section. The bell-shaped end area of the pipe section is placed on the target and seals this area off from the environment. The cooling fluid is guided through the capillary tube and into the bell-shaped end area of the pipe section where it lies in gaseous form and emerges from the outlet end of the capillary tube. As a result of the expansion of the cooling fluid in the bell-shaped area, the cooling fluid is cooled very sharply, so that, depending on the fluid used and on other peripheral conditions and parameters in the bell-shaped end area, the fluid emerging from the capillary tube can either remain gaseous, become liquid or even be solid or gel-like and can hit the target (e.g. the wart) and then evaporate. In this process, the pressure can escape through openings in the wall of the bell-shaped end area.

In another illustrative embodiment of the spray device according to the invention, the applicator has a porous or open-cell body which sits on the pipe section outside the spray head. In the area where the porous or open-cell body sits on the pipe section, said pipe section is provided with through-openings via which the cooling fluid can pass into the porous or open-cell foam body. As a result of the expansion taking place there, the gaseous cooling fluid cools down very sharply and the temperature of the cooling fluid can drop below its boiling point so that it becomes liquid or, depending on the cooling fluid used, can even assume a solid or gel-like aggregate state. In the case of the liquid aggregate state, the cooling fluid "soaks" the porous or open-cell body and then evaporates. In this process, the porous or open-cell body is very sharply cooled and can then be brought very accurately into contact with the target (e.g. a wart).

The spray device can be provided with a pivotable protective cap which can be pivoted between an opened position and a closed position and can be fixed (locked) in the opened position.

Especially in the illustrative embodiment of the spray device according to the invention already described above, with the porous or open-cell body, the pivotable protective cap can be designed in such a way that the fluid outlet valve can be actuated only in the closed state of the protective cap, so that the cooling fluid can pass into the porous or open-cell body made inaccessible by the protective cap. This is intended to prevent children, for example, inadvertently actuating the fluid outlet valve and coming into contact with the porous open-cell body. To do this, they would in fact have to be able to release and pivot the lockable protective cap. For this reason, the spray device is specially designed so that actuation of the fluid outlet valve is impossible with the protective cap opened.

In a further embodiment of the spray device according to the invention, the spray device comprises an adaptor arranged on the outlet end of capillary tube, the adaptor being adapted to be connected to the applicator guiding the cooling fluid to the intended target. In particular, the adaptor may have an essentially pipe-like shape and may being suitable to be moved over the capillary tube. Also, the adaptor may be connected to a finger key to which the valve tappet is mounted in which the capillary tube is arranged. Even more specifically, the adaptor may be connected to the finger key by means of a snap-fit connection. This enables a reliable mounting of the applicator relative to the outlet end of capillary tube, which can be released if desired.

Also, in the afore-mentioned embodiment of the spray device the spray head may comprise a housing having a resilient security element normally preventing the finger key to be actuated. The spray head further may comprise a slider which is arranged such that it can be moved to resiliently deform the said security element so as to allow actuation of the finger key. This embodiment is childproof and prevents the device from being actuated unintentionally.

More specifically, in this embodiment of the spray device the applicator may be adapted and arranged to be pressed against the slider for movement of the slider to resiliently deform the resilient security element so as to allow actuation of the finger key. This is a practical embodiment of a spray device that can be easily handled by adults but cannot be unintentionally actuated by children.

In still a further aspect of the invention, the applicator may comprise an essentially cylindrical portion having slots therein, and wherein the applicator further comprises an annular shield around its outlet opening to prevent areas around the target to be impacted by the cooling fluid. This embodiment provides for a precise deposition of the cooling fluid only to the skin lesion to be treated. Different applicators having outlet openings with different diameters may be provided with the spray device so as to enable the user to select that applicator which suits best for the respective skin lesion to be treated. The annular shield makes sure that the area around the skin lesion is not impacted by the cooling fluid. The slots in the cylindrical portion allow quick evaporation of the film of cooling liquid deposited on the skin lesion to be treated thus providing for an efficient cooling of the cells and increasing efficiency of the treatment of the skin lesion.

A further aspect of the present invention is related to a method for treating skin defects, in which method a cooling fluid is applied to the skin defect to be treated for a period of time sufficient for the cells of the skin defect to be destroyed. In this method, the cooling fluid is sprayed onto the skin defect using a device providing the cooling fluid in gaseous form at an outlet end of a capillary tube. The cooling fluid is sprayed from the outlet end of the capillary tube directly onto the skin defect so as to form a thin film on the skin defect. The thin film then is allowed to evaporate thus supercooling the cells of the skin defect.

Upon being sprayed from the outlet end of the capillary tube the gaseous cooling fluid expands and forms a thin (e.g. liquid) film on the skin defect to be treated (e.g. a wart). It is important, that the film on the skin defect (e.g. the wart) is a thin film, since during evaporation the film evaporates at the surface remote from the wart (i.e. that surface which is not in contact with the wart). On the other hand, in order to efficiently destroy the cells of the skin defect, the low temperature must be provided at the cells of the skin defect. The thicker the film is, the higher is the temperature difference between the surface of the film remote from the skin defect and the surface of the film in contact with the skin defect. For that reason, thin films having a thickness of 0.05 mm to 0.5 mm are preferred. This can be achieved by application only of a predetermined small amount of cooling fluid, e.g. an amount of 25 µl to 250 µl. This can be achieved, for example, through a controlled actuation of the finger key of the spraying device or with the aid of a metering valve. It is thus possible to achieve temperatures of about -50°C or even below at the surface of the skin defect.

In a particularly preferred variant of this method a device is used having an applicator in which the outlet end of the capillary tube is arranged. The applicator is placed on the skin such that it surrounds and seals off the skin defect, and then the cooling fluid is sprayed from the outlet end of the capillary tube directly onto the skin defect. This is a particularly efficient variant of treating the skin defect, since through selection of a suitable inner diameter of the applicator essentially only the cells of the skin defect are affected by the treatment while the tissue surrounding the defect essentially remains unaffected. In addition, the cooling fluid is utilized in a particularly efficient manner.

In a specific variant of this method a device is used having an applicator having an annular shield so as to prevent areas around the skin defect to be treated from being impacted by the cooling fluid sprayed onto the skin defect.

Further advantageous aspects of the invention will become clear from the following description of illustrative embodiments of the spray device according to the invention or of parts thereof. Schematic and/or cross-sectional views are given in the attached drawing, in which:
- Fig. 1: shows a general view of the container, with fluid outlet valve and spray head, in an illustrative embodiment of the spray device according to the invention,
- Fig. 2: shows a perspective cross-sectional view of an illustrative embodiment of a pipe section for connection to the spray head,
- Fig. 3: shows the pipe section from Fig. 2 in an enlarged cross-sectional view,
- Fig. 4: shows the spray head with connected pipe section, and with the porous or open-cell body fitted onto the pipe section (protective cap opened),
- Fig. 5: shows the spray head from Fig. 4, with the protective cap pivoted into the closed position,
- Fig. 6: shows the protective cap in the opened position, to explain the pivotable and lockable nature of the protective cap in the opened position (turned through 180°),
- Fig. 7: shows the protective cap in the closed position after actuation,
- Fig. 8: shows an illustrative embodiment of the bell- shaped end of the pipe section connected to the spray head, in a further illustrative embodiment of the spray device according to the invention,
- Fig. 9: shows an alternative embodiment of the valve tappet for a "male-type" outlet valve,
- Fig. 10: shows an embodiment of the bell-shaped end shown in Fig. 8 surrounding a skin defect to be treated,
- Fig. 11: shows a further embodiment of the spray head of the spray device according to the invention with an applicator being mounted to the spray head,
- Fig. 12: shows the embodiment of Fig. 11 in a rear view,
- Fig. 13: shows a section along line XIII-XIII in Fig. 12,
- Fig. 14: and
- Fig. 15: show perspective views of the finger key of the embodiment of the spray head shown in Fig. 13,
- Fig. 16: shows a longitudinal section through the finger key shown in Fig. 14 and Fig. 15,
- Fig. 17: shows a bottom view of the finger key shown in Fig. 14 and Fig. 15,
- Fig. 18: shows a section along line XIII-XIII in Fig. 17,
- Fig. 19: shows a section along line XIX-XIX in Fig. 17,
- Fig. 20: shows a section along line XX-XX in Fig. 17,
- Fig. 21: shows a perspective view of the valve tappet of the embodiment of the spray head shown in Fig. 13,
- Fig. 22: shows a longitudinal section of the valve tappet shown in Fig. 21, enlarged,
- Fig. 23: shows a perspective view of the slider of the the embodiment of the spray head shown in Fig. 13,
- Fig. 24: shows a bottom view of the slider shown in Fig. 23, enlarged,
- Fig. 25: shows a perspective view of the adaptor of the embodiment of the spray head shown in Fig. 13,
- Fig.: 26 shows a longitudinal section of the adaptor shown in Fig. 25,
- Fig. 27: and
- Fig. 28: show perspective views of the applicator which is connected to the spray head as shown in Fig. 13,
- Fig. 29: shows a top view of the applicator shown in Fig. 27 and Fig. 28,
- Fig. 30: shows a longitudinal section of the applicator shown in Fig. 27 and Fig. 28,
- Fig. 31: and
- Fig. 32: show perspective views of the housing of the embodiment of the spray head shown in Fig. 13,
- Fig. 33: shows a front view of the housing shown in Fig. 31 and Fig. 32, and
- Fig. 34: shows a longitudinal section along line XXXIV- XXXIV in Fig. 33.

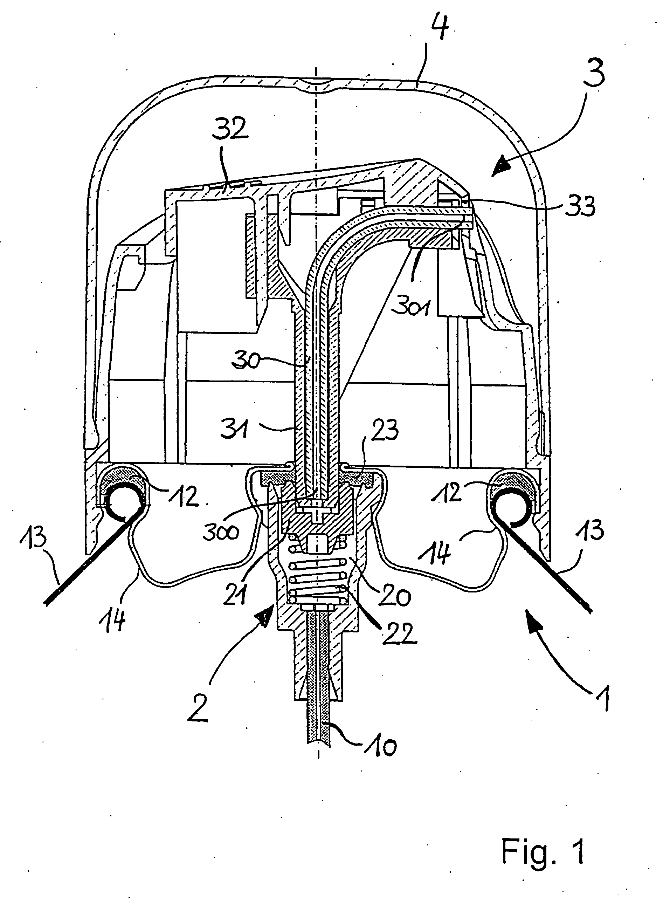

A conventional container 1 is indicated schematically in the general view of some important parts of the spray device according to the invention in Fig. 1. A schematically indicated capillary tube 10 (riser) protrudes into the interior of the container 1, its inlet end extending into the cooling fluid (not shown) which is present in liquid form in the container 1. The outlet end 11 of the capillary tube 10 is connected to the fluid outlet valve 2, and here the end of the capillary tube is received within the fluid outlet valve 2. The fluid outlet valve 2 comprises a valve chamber 20 whose volume is very small and is at any rate less than 50 mm³. Arranged in the inside of the valve chamber 20 there is a valve body 21 which is displaceable counter to the force of a spring 22. A seal 23 seals off the valve chamber 20 and also the interior of the container from the outside. Further seals 12 are provided between the wall 13 and the lid 14 of the container 1.

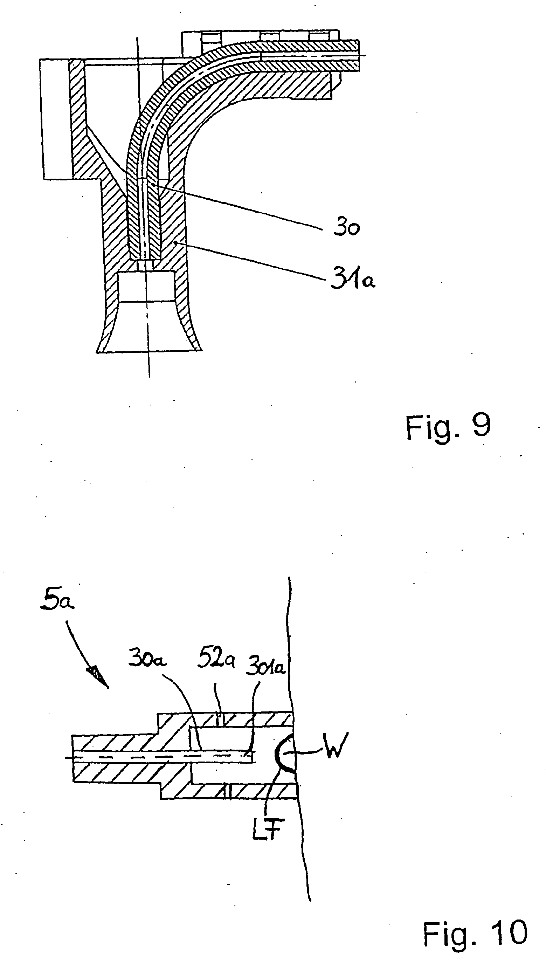

In the spray head 3 there is a further capillary tube 30 whose inlet end 300 is arranged in a valve tappet 31. The valve tappet 31 is in engagement with the valve body 21. While in Fig. 1 a "female"-type outlet valve 2 is shown, "male"-type outlet valves are also possible. The valve tappet 31a may then be designed in the manner shown in Fig. 9, while the other elements may remain unchanged. When the finger key 32 is actuated, the valve tappet 31 and consequently the valve body 21 are moved downward counter to the force of the spring 22, as a result of which the fluid outlet valve 2 is opened. The cooling fluid flows out of the container 1 through the capillary tube 10, where it already forms an aerosol and further evaporates, then into the valve chamber 20, and through the latter into the inlet end 300 of the capillary tube 30 in the spray head 3. At the outlet end 301 of the capillary tube 30 from the spray head, said spray head 3 has an opening 33 into which a pipe section can be inserted (as will be explained in more detail below). Fig. 1 also shows a protective cap 4. The way in which this protective cap can be designed, and its purpose, will be explained in more detail below.

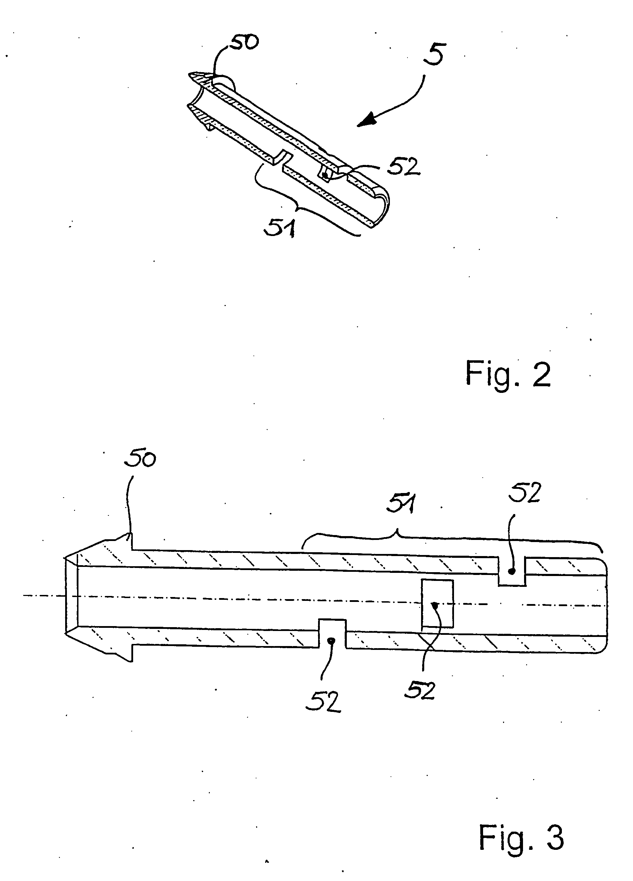

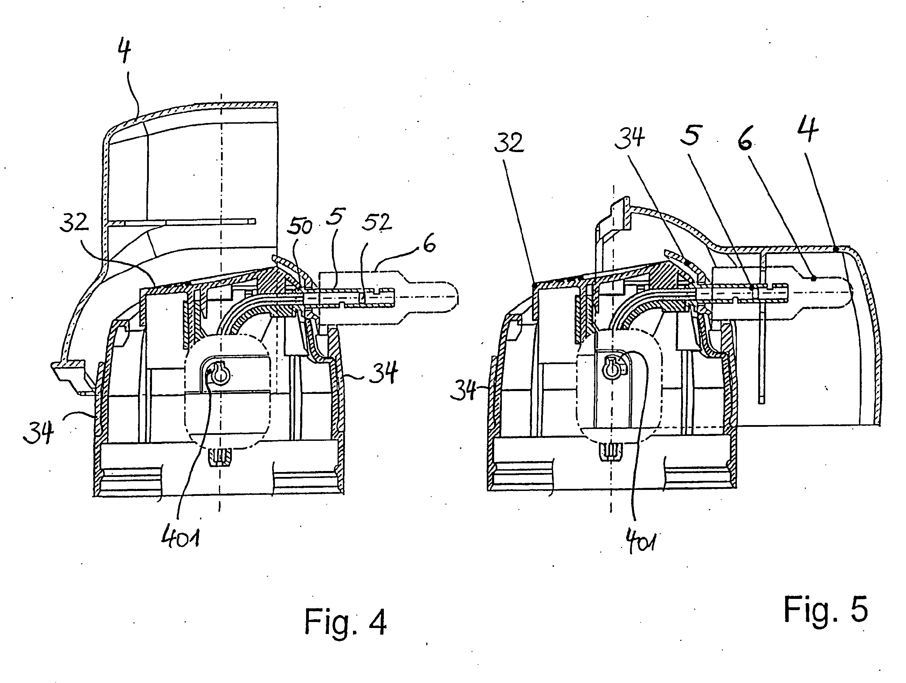

As has already been mentioned, a pipe section can be inserted into the opening 33 in the spray head 3. The manner in which such a pipe section 5 can be designed, for example, is shown in the form of an illustrative embodiment in a perspective cross-sectional view in Fig. 2 and in an enlarged cross-sectional view in Fig. 3. A locking projection 50 at one end of the pipe section 5 can be guided through the opening 33 in the spray head 3, the locking projection 50 engaging in the manner of a snap-fit connection and then being joined to the spray head 3 (see also Fig. 4 and Fig. 5). It will also be seen that the pipe section 5 is provided, along an area 51, with through-openings 52 in its wall.

The pipe section 5 is part of an applicator in which the cooling fluid is guided toward an intended target, for example toward a skin defect that is to be removed (e.g. a wart). The applicator in this case comprises, in addition to the pipe section 5, also a porous or open-cell body 6 (shown in Fig. 4 and Fig. 5 by a broken line) which sits on the pipe section 5, specifically at least in the area 51 in which the through-openings 52 are provided in the wall of the pipe section 5. The pipe section 5 can be made, for example, from a suitable plastic such as polypropylene (PP) or polyethylene (PE), or of Teflon, nylon or POM (polyoxymethylene).

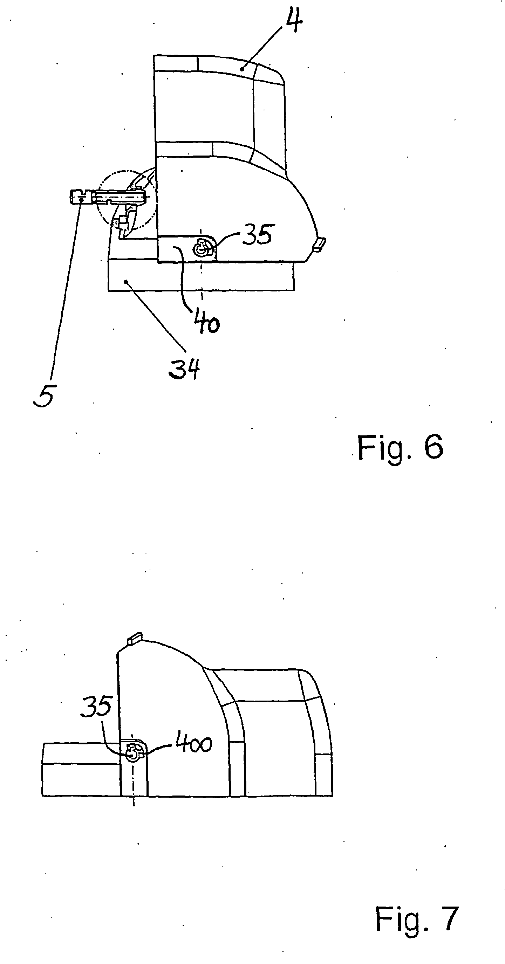

Fig. 4 now shows the spray head 3 and also the applicator which comprises the pipe section 5 connected to it and the porous or open-cell body 6 (shown by broken lines) sitting on the pipe section 5. This body 6 can, for example, be made from an open-cell foam of polyurethane (PU), polypropylene (PP) or polyethylene (PE), or of cellulose or gauze. The protective cap 4 is shown in the opened position in Fig. 4 and can be fixed (e.g. locked) in this opened position. Fig. 4 also shows a safety ring 34 on the spray head 3. Together with the specially designed protective cap 4, this safety ring 34 has the effect that, when the protective cap 4 is in the opened position, the finger key 32 is not accessible and, therefore, the fluid outlet valve 2 cannot be actuated (childproof feature). The corresponding mechanism for pivoting the protective cap 4 into the closed position, shown in Fig. 5, is already indicated in Fig. 4 and Fig. 5 within a broken-line area, but it is explained in greater detail below with reference to Fig. 6 and Fig. 7.

To explain the mechanism for pivoting the protective cap 4, Fig. 6 is a schematic representation of the protective cap 4 and the safety ring 34, the protective cap 4 being shown in the opened position, and the pipe section 5 can also be seen (but without a porous or open-cell body fitted onto it). The view in Fig. 6 is also turned 180° about the longitudinal axis, as can be seen from the fact that the pipe section 5 is pointing to the left instead of to the right. It will be noted that the protective cap 4 has an "activation area" 40 which is flexible to some extent and which is correspondingly also present on the opposite side (not visible in Fig. 6). In this activation area 40, a slit-shaped passage 400 is provided into which a positionally fixed locking element 35 protrudes. Without any additional measure, the locking element thus prevents pivoting of the protective cap 4 from this opened position, and instead the latter is locked in the opened position because the locking element 35 is as it were held "captive" in the slit-shaped passage 400.

To pivot the protective cap 4, the user presses simultaneously from both sides (in Fig. 6 from front and from behind) against the activation area 40, by which means the locking element 35 passes completely through the slit 400 and the protective cap 4 can be pivoted. The user pivots the protective cap until it reaches the closed position shown in Fig. 7. During the pivoting, the locking element 35 slides in a corresponding groove-like recess 401 (not a passage), see Fig. 4 and Fig. 5, until it reaches the closed position. It is also quite conceivable to provide a corresponding further slit-shaped passage which allows the protective cap 4 also to be locked in the closed position according to Fig. 7.

For treating a skin defect (e.g. a wart), the user first moves the protective cap 4 from the opened position to the closed position, as has been described with reference to Fig. 6 and Fig. 7, that is to say from the position according to Fig. 4 to the position according to Fig. 5. In the closed position according to Fig. 5, the user now presses on the finger key 32, by which means the fluid outlet valve 2 (Fig. 1) is opened and the cooling fluid passes through the capillary tube 10 (riser tube), where an aerosol formation already takes place and it then further evaporates, into the valve chamber 20 and then into the capillary tube 30. In the further course of its travel, the now gaseous cooling fluid passes through the pipe section 5, and in particular through the latter's through-openings 52 and the outlet end of the pipe section 5, into the porous or open-cell body 6. As a result of the expansion taking place there, the cooling fluid cools to below its boiling point and can become liquid (or, depending on the cooling fluid used and on the other conditions or parameters, even solid or gel-like) and "soak" the porous or open-cell body 6. In the subsequent evaporation of the cooling fluid of the "soaked" body 6, the porous or open-cell body 6 cools very sharply (e.g. below a temperature of -50°C) and, for treatment purposes, can then be brought into contact with the exact target area of the skin defect to be treated (e.g. a wart).

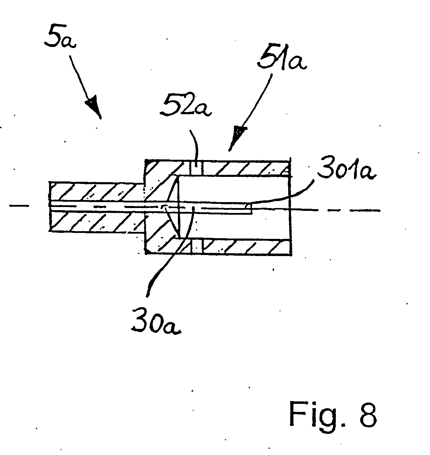

Fig. 8 shows the outlet end of a further illustrative embodiment of the pipe section 5a. Here, the applicator comprises only the pipe section 5a which, at its free end, has a bell-shaped end area 51a. The capillary tube 30a (not shown in cross section here) is longer in this illustrative embodiment than in the previously described illustrative embodiment with the porous or open-cell body; the outlet end 301a of the capillary tube 30a here protrudes into the bell-shaped end area 51a. In the wall of the bell-shaped end area 51a, through-openings 52a are provided (e.g. four of them, each offset by 90° about the circumference, of which two can be seen in Fig. 8) through which the pressure is able to escape.

The pipe section 5a can be made in particular of polypropylene, polyethylene, a metal, PON, Teflon, glass or ceramic, or of any other sufficiently stable plastics.

The mode of operation is as follows. The spray device is actuated in the same way as in the previously described illustrative embodiment, although the protective cap 4 may, for example, be designed so as to be completely removable (as shown in Fig. 1 for example) and, if appropriate, another kind of childproof safety feature can be provided. The bell-shaped end area 51a of the pipe section 5a is placed on the skin in such a way that it surrounds and seals off the skin defect to be treated (e.g. the wart). When the finger key 32 is operated, the cooling fluid is conveyed in the gaseous state to the outlet end 301a of the capillary tube 30a. As a result of the expansion taking place in the bell-shaped area 51a, the cooling fluid cools very sharply, for example to below its boiling point, so that it strikes the skin defect in the gaseous state, in liquid form (or, depending on the cooling fluid used and on the other conditions or parameters) and then evaporates. The skin defect (e.g. the wart) is greatly cooled and can thus be destroyed.

For example, at the time the gaseous cooling liquid is sprayed from the outlet end 301a of the capillary tube 30a it may form an aerosol which is deposited as a thin liquid film LF on the skin defect W (e.g. the wart), as this is shown in Fig. 10. It is especially advantageous to obtain a thin liquid film LF on the skin defect W having a thickness of between 0.05 mm an 0.5 mm to obtain a particularly effective cooling. This is so because the liquid film LF - evaporates at the surface remote from the skin defect W (i.e. at the surface not contacting the skin defect W). Consequently, the thicker the liquid film LF is the higher is the difference between the temperature of the liquid film LF at the evaporation surface and the temperature of the liquid film LF at the surface in contact with the skin defect W. Film thicknesses in the afore-mentioned range can be achieved by spraying an amount of cooling fluid of between 25µl and 250µl. This can be achieved, for example, through a controlled actuation of the finger key 32 of the spray device (which is possible due to the low mass flow through the capillary tube 30a) or with the aid of a metering valve setting the amount of cooling fluid to be metered per spray burst. Such metereing valves are available on the market. It is thus possible to achieve temperatures of about -50°C or even below at the surface of the skin defect W with small amounts of cooling fluid.

The above-described illustrative embodiments of the spray device according to the invention were described for the field of application of treatment of skin defects. The capillary tubes have an internal diameter in the range of 0.35 mm to 1 mm. Another reduction nozzle for additional reduction of the required amount of cooling fluid could even have an internal diameter of less than 0.2 mm. Examples of coolants that can be used are dimethyl ether, propane, isobutane, n-butane, propellant gases 134A, 157 or 227, or CO₂. The container pressure can, for example, be 12 bar at 50°C. The temperatures that can be attained during treatment lie in particular in the range of -25°C to -55°C and are therefore in some cases quite considerably below the otherwise customary temperatures, which lie just below the boiling point of -25°C.

However, the spray device according to the invention is not limited to the field of application of treatment of warts or skin defects, and instead it is generally suitable for applications in which precisely targeted and very efficient cooling is important.

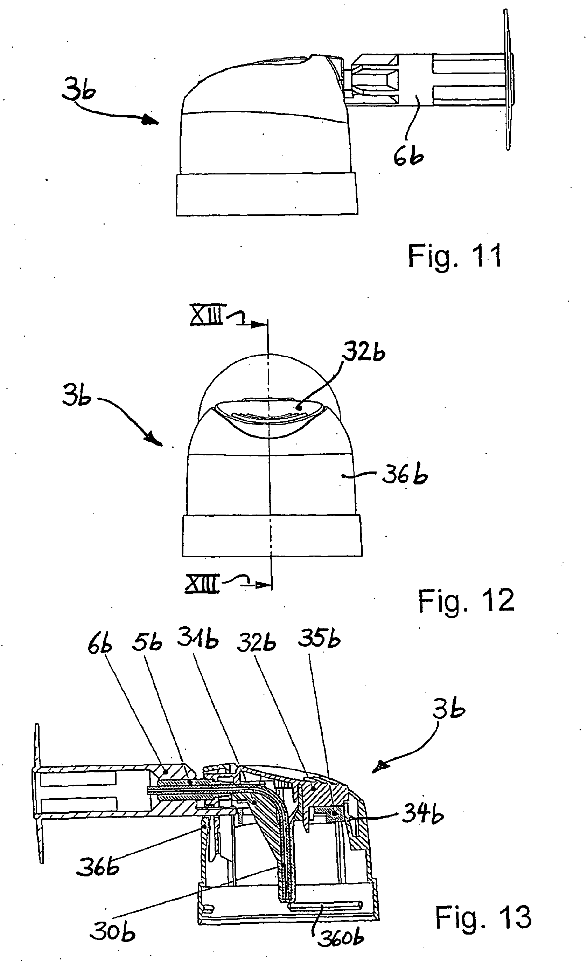

Another embodiment of the spray head of the spray device according to the invention will now be described with the aid of Figs. 11-34, which show the spray head and its components both in an assembled state (Figs. 11-13) as well as in a dissassembled state where only the single components are represented.

Fig. 11 shows the spray head 3b in the assembled state in a side view with an applicator 6b being attached to the spray head 3b in a manner that will be described in more detail below. Fig. 12 shows the assembled spray head 3b in a rear view in which a finger key 32b can be seen in greater detail. Finally, Fig. 13 shows a longitudinal section (see line XIII-XIII in Fig. 12) through the spray head 3b in the assembled state.

From Fig. 13 it can be seen that valve tappet 31b carrying capillary tube 30b is mounted to spray head 3b. More particularly, valve tappet 31b is attached to finger key 32b, as will be described in more detail below. A slider 35b is provided that can be moved in the axial direction (in Fig. 13 to the right) so as to act upon a resilient security element 34b (lock spring) provided in housing 36b. Thus, it is ensured that finger key 32b cannot be unintentionally pressed prior to deforming resilient security element (lock spring). Only then downward pressing of finger key 32b is possible and, accordingly, the spray-head is "child-resistant". At the outlet opening 33b a pipe-like adaptor 5b is provided onto which applicator 6b is mounted. The assembled spray head .3b shown in Fig. 13 can be mounted onto a container containing a cooling fluid with the aid of projecting ribs 360b provided on the interior wall of housing 36b of spray head 3b in a manner similar to that already shown enlarged in Fig. 1.

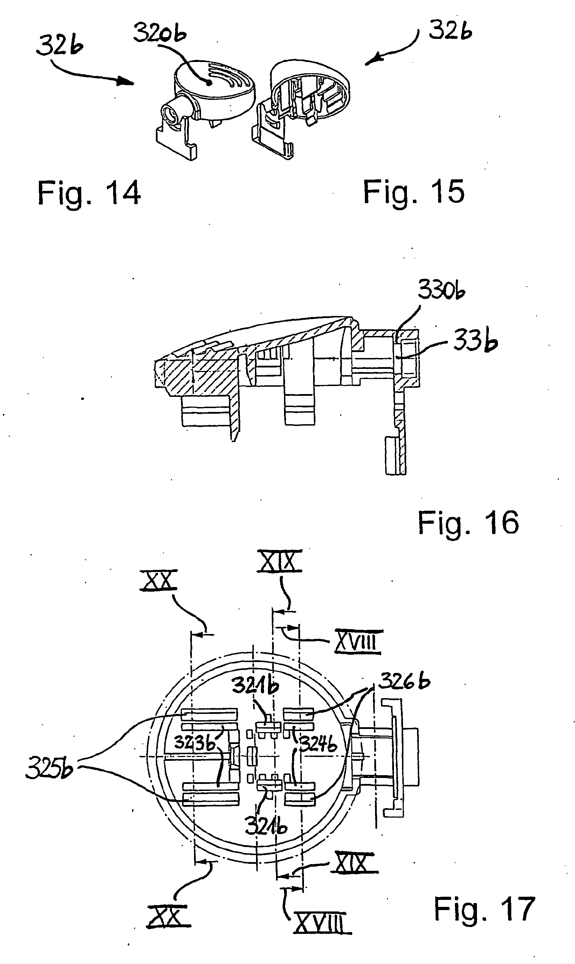

Fig. 14 and Fig. 15 show perspective views of finger key 32b, Fig. 16 shows a longitudinal section through finger key 32b, Fig. 17 shows a bottom view of finger key 32b, and Figs.. 18-20 show respective sectional views along the respective lines of Fig. 17. The overall shape of finger key 32b can be seen best in the perspective views of finger key 32b as shown Fig. 14 and Fig. 15. Finger key 32b comprises a comparatively large actuation zone 320b, which is to be pressed downwards by the user in order to actutate the spray device. As is indicated in Fig. 14, a plurality of latches and guiding elements are provided at the underside of finger key 32b and are projecting downwardly from the underside of finger key 32. The function of these latches and guiding elements will be discussed in more detail below.

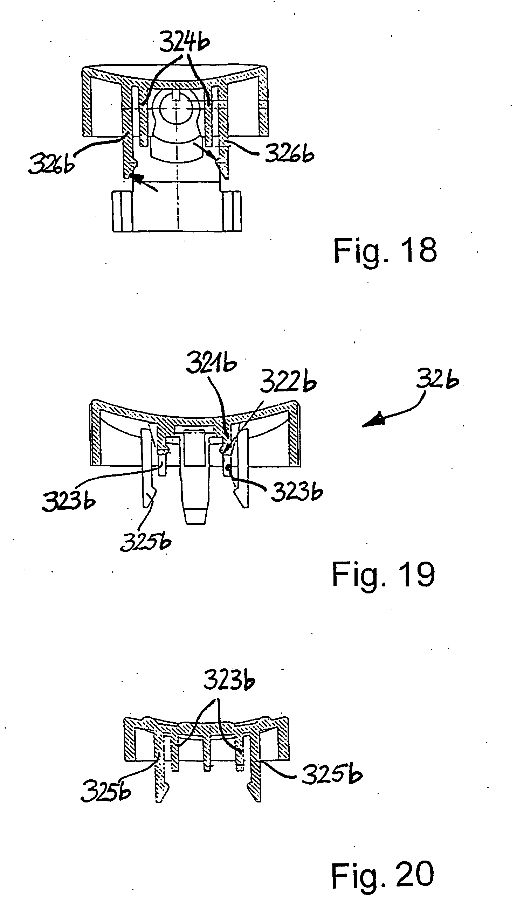

A first pair of latches 321b is arranged close to the censer of finger key 32b, as is shown in Fig. 16. Latches 321b are each provided with an inwardly protruding rib 322b (see Fig. 18) so as to enable a "click-in" of valve tappet 31b carrying capillary tube 30b. To provide for proper orientation of valve tappet 31b during and after "clicking-in", pairs of guiding elements 323b and 324b are provided respectively.

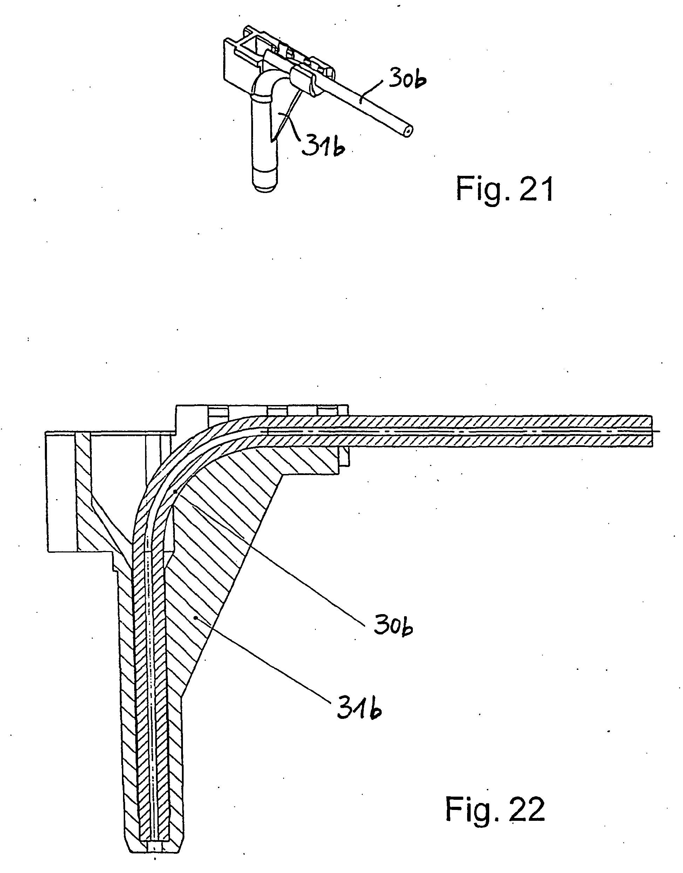

Valve tappet 31b carrying capillary tube 30b is shown in perspective view in Fig. 21, while Fig. 22 shows an enlarged longidutinal section through valve tappet 31b and capillary tube 30b.

To assemble valve tappet 31b and finger key 32b, capillary tube 30b is first guided through opening 33b and then valve tappet 31b is "clicked-in" from below, so that valve tappet 31 is securely held in place against falling down by latches 321b, and against axial displacement by an abutment surface 310b that abuts against a corresponding abutment surface provided for in finger key 32 (as can be seen, for example, in Fig. 17).

Once valve tappet 31b carrying capillary tube 30b has been "clicked-in", the next step is to assemble slider 35b to the above-described pre-assembled finger key / valve tappet assembly.

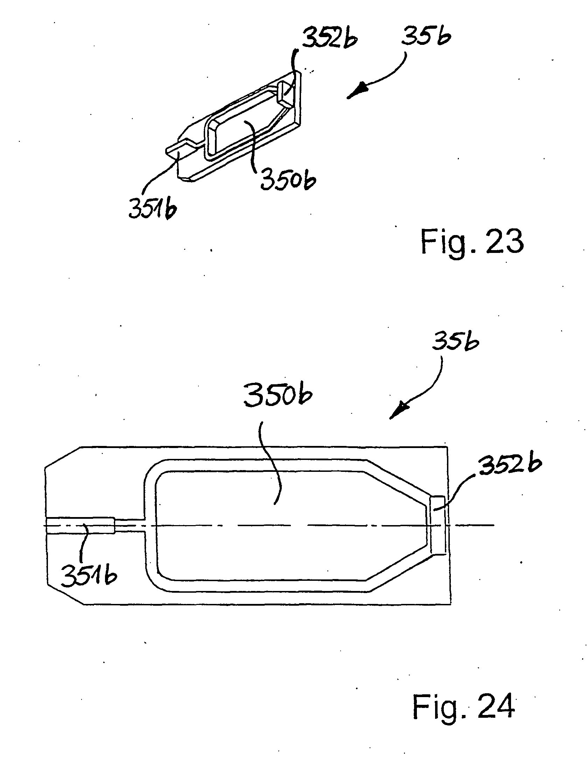

Slider 35b is shown in perspective view in Fig. 23 while Fig. 24 shows an enlarged bottom view of slider 35b. Slider 35b generally is an essentially small flat plate-shaped element comprising a relatively large longitudinally extending opening 350b. At its rear end slider 35b has a downwardly projecting fin 351b which is intended to act against resilient security element 34b (lock spring) in order to allow finger key 32b to be pressed downwards. At its front end slider 35b has a downwardly projecting abutment element 352b that can be acted upon by applicator 6b so as to move slider 35 axially backwards and to deform resilient security element 34b so as to allow finger key 32b to be pressed.

To assemble slider 35b to the pre-assembled finger key / valve tappet assembly, slider 35b is "clicked-in" from below. Slider 35 is assembled such that its opening 351b extends around valve tappet 31b. For the "clicking-in" of slider 35b, finger key 32 comprises two pairs of latches 325b and 326b which can be seen in Fig. 17 and in Figs. 18-20, respectively. Latches 325b and 326b are each provided with an inwardly protruding rib 327b or 328b, respectively, so as to allow the "click-in" of slider 35b. The pre-assembly now comprises finger key 32b, valve tappet 31b carrying capillary tube 30b, and slider 35b.

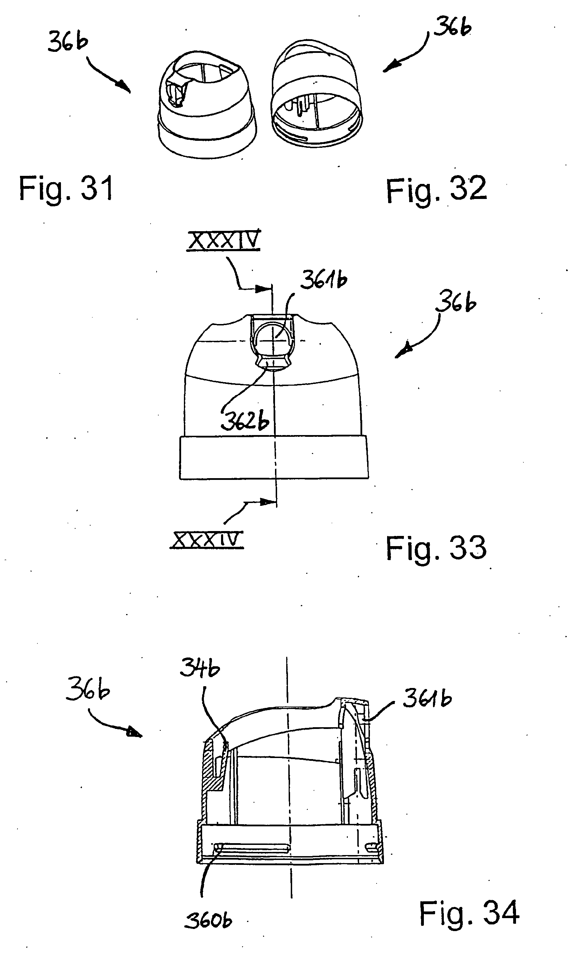

This pre-assembly comprising finger key 32b, valve tappet 31b carrying capillary tube 30b, and slider 35b is now inserted into housing 36b from below so as to enable the flexible outlet portion of capillary tube 30b to be assembled through opening 361b of housing 36b (see Fig. 34).

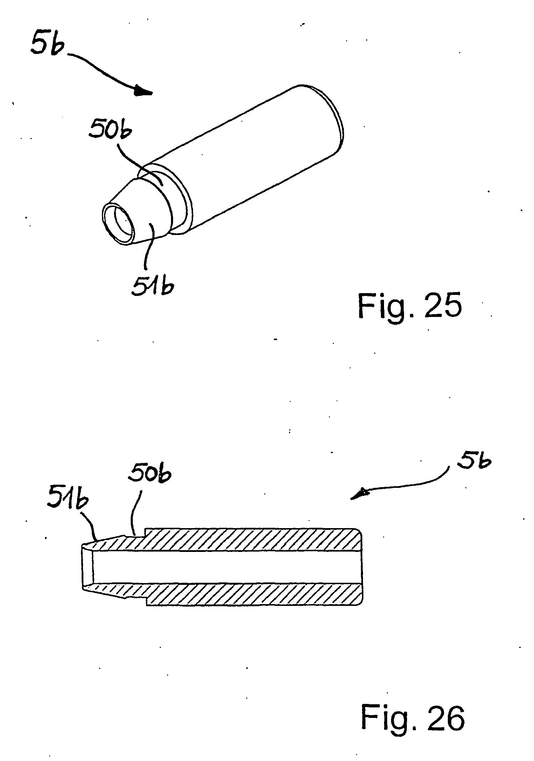

The next step then is to mount pipe-like adaptor 5b shown in Fig. 25 in a perspective view, while Fig. 26 shows a longitudinal section through adapter 5b. In order to mount adaptor 5b, it is moved axially towards housing 36b and finger key 32b over the outlet end of capillary tube 30b projecting through opening 361b of housing 36b. Adaptor 5b has a recess 50b and a tapered portion 51b. Finger key 32b has an inwardly projecting rim 330b (see Fig. 16). As adaptor 5b is axially moved' towards housing 36b or finger key 32b, respectively, over the outlet end of capillary tube 30b tapered portion 51b passes rim 330b, and rim 330b snaps into recess 50b of adaptor 5b thus mounting adaptor 5b to finger key 32b through a snap-fit connection.

Housing 36b is shown in perspective view in Fig. 31 and Fig. 32, while Fig. 33 shows a front view of housing 36b and Fig. 34 shows a section along line XXXIV-XXXIV in Fig. 33. In Fig. 34 the resilient security element (lock spring) 34b and the ribs 361b for mounting the housing (or spray head, respectively) to the container containing the cooling fluid can be seen best.

When the pre-assembly described above has been mounted to housing 36b the spray head 3b is then complete. For an application of the cooling fluid to the skin, however, in this embodiment applicator 6b is to be mounted to spray head 3b.

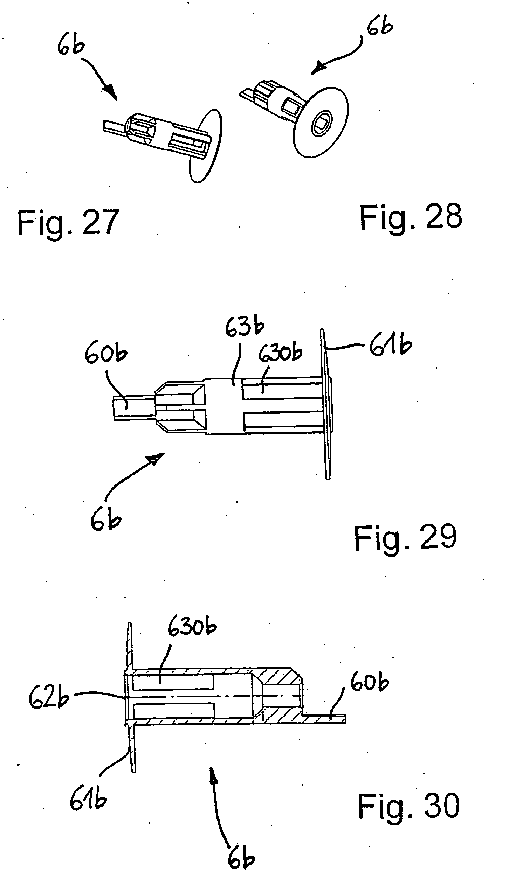

Applicator 6b is shown in Fig. 27 and Fig. 28 in perspective view, while Fig. 29 shows a top view of applicator 6b and Fig. 30 shows a longitudinal section through applicator 6b. At one end applicator 6b has an extension 60b while at the other end it is provided with an annular shield 61b around an opening 62b through which the cooling fluid is to impinge on a skin lesion to be treated so as to form a thin film of cooling fluid on the skin lesion. Further, applicator 6b has a cylindrical portion 63b being provided with slots 630b serving for the evaporation of the fluid that has been deposited on the skin lesion.

Applicator 6b is to be moved axially over adaptor 5b until extension 60b contacts abutment element 352b of slider 35b. In order to allow insertion of extension 60b of applicator 6b, housing 36b is provided with a correspondingly formed opening 362b (see Fig. 33).

Once applicator 6b has been mounted over adaptor 5b in the manner described above, the spray device is ready to be used (see Fig. 13 and imagine, that the assembly shown in Fig. 13 has been mounted to a container in a manner similar to that shown in Fig. 1. For the treatment of a skin lesion, e.g. a wart, opening 62b of applicator 6b is arranged over the wart with the annular shield surrounding the region around the wart so as to prevent healthy skin to be impacted by the cooling fluid. Of course, it is possible to provide a number of similar applicators 6b having different diameters of opening 62b so as to be able to treat skin lesions of different size and still only contact the area of the skin where the skin lesion to be treated is located. Turning back to Fig. 13, when applicator 6b has been positioned with opening 62b (Fig. 30) being positioned around the skin lesion, the spray device is slightly pressed in the direction towards the skin lesion. As a consequence, extension 60b (Fig. 30) is pressed against abutment element 352b of slide 35b thus moving slider 35b backwards against resilient security element 34b (lock spring). Having deformed resilient security element 34b (lock spring) to a certain extent it is then possible to press finger key 32b downwards thus actuating the valve and allowing cooling fluid to be sprayed directly and precisely onto the skin lesion, as this has already been described further above. After having completed the spraying process, finger key 32 is no longer pressed by the user and moves upwardly again until resilient security element 34b (lock spring) again locks finger key 32b, so that the spray device cannot unintentionally be actuated, e.g. by children.

Finally, it is to be mentioned that in Fig. 1 only a valve 2 having a closed valve body 21 is shown, so that upon using the spray device in an upside-down position the mass flow (percentage of droplets contained in the flow of cooling fluid) is less than if the device were used in the upright position since in the upside-down position only the gaseous phase of the cooling fluid contained in container 1 flows through the capillary tube 10, since the inlet end of capillary tube 10 (riser) then only extends into the gaseous phase. In order to overcome a dispensing of pure gaseous phase cooling fluid out of container 1 in such cases, the valve body 21 may be provided with a small through-bore connecting the inner space of container 1 with valve chamber 20. Accordingly, regardless of whether the spray device is used in an upright position or in an upside-down position a mixture of liquid and gaseous phase of cooling fluid is dispensed out of ontainer 1. This results in a slightly thinner film on the skin lesion to be treated, however, a thinner film may even increase the efficiency of the treatment process, since it is only that layer of the film which is in direct contact with the skin lesion that effects the destruction of the cells. Consequently, a thicker film of cooling fluid deposited on the skin lesion does not necessarily improve the process of treatment of the skin lesion (rather, efficiency of the cooling process depends on the overall thickness of the film).

## Claims

1. A spray device comprising a reservoir (1) in which a cooling fluid is stored in liquid form at an overpressure, with a fluid outlet valve (2), and with a spray head (3; 3b) which is mounted on the reservoir (1) and has a capillary tube (30; 30a; 30b) arranged in a valve tappet (31; 31b) in such a way that the inlet end (300) of the capillary tube (30; 30a; 30b) is connected to the fluid outlet valve (2) of the reservoir (1) and causes the cooling fluid to pass into the capillary tube (30; 30a; 30b) when the fluid outlet valve (2) is actuated, wherein the reservoir (1) contains a further capillary tube (10) whose inlet end extends into the cooling fluid stored in liquid form in the reservoir (1), and whose outlet end is connected to the fluid outlet valve (2), **characterised in that** the internal diameter of both the capillary tube (30; 30a; 30b) and the further capillary tube (10) is in the range of 0.35 mm to 1 mm, and **in that** the cooling fluid is provided in gaseous form at the outlet end of the capillary tube (30; 30a, 30b).

2. The spray device as claimed in claim 1, in which the spray head (3; 3b) is provided with a receiver opening (33; 361b) for receiving an applicator (5, 6; 5a; 6b) in which the fluid is guided to an intended target.

3. The spray device as claimed in claim 2, in which the applicator has a pipe section (5; 5a) which is firmly connected to the spray head (3).

4. The spray device as claimed in claim 3, in which the pipe section (5; 5a) is connected to the spray head (3) by means of a snap-fit connection.

5. The spray device as claimed in anyone of claims 3 or 4, in which the pipe section (5a) has, at its free end, a bell-shaped end area (51a) whose internal diameter is greater than the internal diameter of the remaining pipe section (5a), and in which the capillary tube (30a) of the spray head (3) is guided through the pipe section (5a), and its outlet end (301a) is arranged in the bell-shaped end area (51a) of the pipe section (5a).

6. The spray device as claimed in anyone of claims 3 or 4, in which the applicator has a porous or open-cell body (6) which sits on the pipe section (5) outside the spray head (3), and in which the pipe section (5), in the area (51) where the porous or open-cell body (6) sits on the pipe section (5), is provided with through-openings (52) via which the cooling fluid can pass into the porous or open-cell foam body (6).

7. The spray device as claimed in anyone of claims 1 to 5, in which a pivotable protective cap (4) is provided which can be pivoted between an opened position and a closed position and can be fixed (35, 400) in the opened position.

8. The spray device as claimed in claim 6 and claim 7, in which the pivotable protective cap (4) is designed in such a way that, in the closed state of the protective cap (4), the fluid outlet valve (2) can be actuated so that the cooling fluid can pass into the porous or open-cell body (6) made inaccessible by the protective cap (4).

9. The spray device as claimed in claim 2, wherein the spray device comprises an adaptor (5b) arranged on the outlet end of capillary tube (30b), the adaptor (5b) being adapted to be connected to the applicator (6b) guiding the cooling fluid to the intended target.

10. The spray device as claimed in claim 9, wherein the adaptor (5b) has an essentially pipe-like shape, the adaptor (5b) being suitable to be moved over the capillary tube (30b) and to be connected to a finger key (32b) to which the valve tappet (31b) is mounted in which the capillary tube (30b) is arranged.

11. The spray device as claimed in claim 10, wherein the adaptor (5b) is connected to the finger key (32b) by means of a snap-fit connection (50b,330b).

12. The spray device as claimed in claim 10 or claim 11, wherein the spray head (3b) comprises a housing (36b) having a resilient security element (34b) normally preventing the finger key (32b) to be actuated, and wherein the spray head (3b) further comprises a slider (35b) being arranged such that it can be moved to resiliently deform the security element (34b) so as to allow actuation of the finger key (32b).

13. The spray device as claimed in claim 12, wherein the applicator (6b) is adapted and arranged to be pressed against the slider (35b) for movement of the slider (35b) to resiliently deform the resilient security element (34b) so as to allow actuation of the finger key (32b).

14. The spray device according to any one of claims 9 to 13, wherein the applicator (6b) comprises an essentially cylindrical portion (63b) having slots (630b) therein, and wherein the applicator (6b) further comprises an annular shield (61b) around its outlet opening (62b) to prevent areas around the target to be impacted by the cooling fluid.

15. A method for treating skin defects, in which method a cooling fluid is applied to the skin defect to be treated for a period of time sufficient for the cells of the skin defect to be destroyed, wherein the cooling fluid is sprayed onto the skin defect using a device providing the cooling fluid in gaseous form at an outlet end of a capillary tube and wherein the cooling fluid is sprayed from the outlet end of the capillary tube directly onto the skin defect so as to form a thin film on the skin defect, and wherein the thin film then is allowed to evaporate thus supercooling the cells of the skin defect.

16. Method according to claim 15, wherein a device is used having an applicator in which the outlet end of the capillary tube is arranged, and wherein the applicator is placed on the skin such that it surrounds the skin defect, and wherein the cooling fluid is then sprayed from the outlet end of the capillary tube directly onto the skin defect.

17. A method according to claim 16, wherein a device is used having an applicator having an annular shield so as to prevent areas around the skin defect to be treated from being impacted by the cooling fluid sprayed onto the skin defect.

## Patentansprüche

1. Sprühvorrichtung umfassend einen Vorratsbehälter (1), in welchem ein Kühlfluid in flüssiger Form unter Überdruck bereitgestellt wird, mit einem Fluid-Austrittsventil (2), sowie mit einem auf dem Vorratsbehälter (1) aufgesetzten Sprühkopf (3; 3b), der eine Kapillare (30; 30a; 30b) aufweist, die in einem Ventilstössel (31; 31b) derart angeordnet ist, dass das Eintrittsende (300) der Kapillare (30; 30a; 30b) an das Fluid-Austrittsventil (2) des Vorratsbehälters (1) anschliesst und bei einer Betätigung des Fluid-Austrittsventils (2) das Eintreten des Kühlfluids in die Kapillare (30; 30a; 30b) bewirkt, wobei im Vorratsbehälter (1) eine weitere Kapillare (10) angeordnet ist, deren Eintrittsende sich in das in flüssiger Form im Vorratsbehälter (1) bereitsgestelle Kühlfluid hinein erstreckt, und deren Austrittsende mit dem Fluid-Austrittsventil (2) verbunden ist, **dadurch gekennzeichnet, dass** der Innendurchmesser der Kapillare (30; 30a; 30b) und der weiteren Kapillare (10) im Bereich von 0.35 mm bis 1 mm liegt, und dass das Kühlfluid am Austrittsende der Kapillare (30; 30a, 30b) gasförmig bereitgestellt wird.

2. Sprühvorrichtung nach Anspruch 1, in welcher der Sprühkopf (3;3b) mit einer Aufnahmeöffnung (33; 361b) versehen ist zur Aufnahme eines Applikators (5, 6; 5a; 6b), in welchem das Fluid zu einem zu beaufschlagenden Ziel geführt wird.

3. Sprühvorrichtung nach Anspruch 2, bei welcher der Applikator ein Rohrstück (5; 5a) aufweist, welches mit dem Sprühkopf (3) fest verbunden ist.

4. Sprühvorrichtung nach Anspruch 3, bei welcher das Rohrstück (5; 5a) mittels einer Schnappverbindung mit dem Sprühkopf (3) verbunden ist.

5. Sprühvorrichtung nach einem der Ansprüche 3 oder 4, bei welcher das Rohrstück (5a) an seinem freien Ende einen glockenförmig ausgebildeten Endbereich (51a) aufweist, dessen Innendurchmesser grösser ist als der Innendurchmesser des übrigen Rohrstücks (5a), und bei welcher die Kapillare (30a) des Sprühkopfs (3) durch das Rohrstück (5a) hindurch geführt ist und ihr Austrittsende (301a) im glockenförmig ausgebildeten Endbereich (51a) des Rohrstücks (5a) angeordnet ist.

6. Sprühvorrichtung nach einem der Ansprüche 3 oder 4, bei welcher der Applikator einen porösen oder offenporigen Körper (6) aufweist, der ausserhalb des Sprühkopfs (3) auf dem Rohrstück (5) aufsitzt, und bei welcher das Rohrstück (5) in dem Bereich (51), in welchem der poröse oder offenporige Körper (6) auf dem Rohrstück (5) aufsitzt, mit Durchtrittsöffnungen (52) versehen ist, durch die das Kühlfluid hindurch in den porösen oder offenporigen Schaumstoffkörper (6) gelangen kann.

7. Sprühvorrichtung nach einem der Ansprüche 1 bis 5, bei welcher eine schwenkbare Schutzkappe (4) vorgesehen ist, die zwischen einer geöffneten und einer geschlossenen Stellung schwenkbar und in der geöffneten Stellung feststellbar ist (35,400).

8. Sprühvorrichtung nach Anspruch 6 und Anspruch 7, bei welcher die schwenkbare Schutzkappe (4) so ausgebildet ist, dass im geschlossenen Zustand der Schutzkappe (4) das Fluid-Austrittsventil (2) betätigbar ist, sodass das Kühlfluid in den durch die Schutzkappe (4) unzugänglichen porösen oder offenporigen Körper (6) gelangen kann.

9. Sprühvorrichtung nach Anspruch 2, wobei die Sprühvorrichtung einen am Austrittsende der Kapillare (30b) angeordneten Adapter (5b) umfasst, wobei der Adapter (5b) so ausgebildet ist, dass er mit dem Applikator (6b), welcher das Kühlfluid zum zu beaufschlagenden Ziel führt, verbindbar ist.

10. Sprühvorrichtung nach Anspruch 9, wobei der Adapter (5b) eine im wesentlichen rohrartige Form hat, wobei der Adapter (5b) geeignet ist, über die Kapillare (30b) bewegt und mit einem Fingertaster (32b) verbunden zu werden, auf dem der Ventilstössel (31b) mit der darin angeordneten Kapillare (30b) aufgesetzt ist.

11. Sprühvorrichtung nach Anspruch 10, wobei der Adapter (5b) mittels einer Schnappverbindung (50b,330b) mit dem Fingertaster (32b) verbunden ist.

12. Sprühvorrichtung nach Anspruch 10 oder Anspruch 11, wobei der Sprühkopf (3b) ein Gehäuse (36b) umfasst mit einem federnden Sicherheitselement, (34b) das normalerweise die Betätigung des Fingertasters (32b) verhindert, und wobei der Sprühkopf (3b) ferner einen Schieber (35b) umfasst, der so angeordnet ist, dass er so bewegt werden kann, dass er das Sicherheitselement (34b) elastisch verformt, um so die Betätigung des Fingertasters (32b) zu ermöglichen.

13. Sprühvorrichtung nach Anspruch 12, wobei der Applikator (6b) so ausgebildet und angeordnet ist, dass er zur Bewegung des Schiebers (35b) zur elastischen Verformung des federnden Sicherheitselements (34b) gegen den Schieber (35b) gedrückt werden kann und so die Betätigung des Fingertasters (32b) erlaubt.

14. Sprühvorrichtung nach einem der Ansprüche 9 bis 13, wobei der Applikator (6b) einen im wesentlichen zylindrischen Teil (63b) mit darin angebrachten Schlitzen (630b) umfasst, und wobei der Applikator (6b) ferner einen ringförmigen Schild (61b) um die Austrittsöffnung (62b) herum umfasst, um zu verhindern, dass Gebiete um das Zielgebiet herum mit Kühlfluid beaufschlagt werden.

15. Verfahren zur Behandlung von Hautdefekten, bei welchem Verfahren ein Kühlfluid auf den zu behandelnden Hautdefekt aufgebracht wird für eine Zeitdauer, welche eine Zerstörung der Zellen des Hautdefekts zur Folge hat, wobei das Kühlfluid mittels einer Vorrichtung, die das Kühlfluid am Austrittsende einer Kapillare gasförmig bereitstellt, auf den Hautdefekt aufgesprüht wird, und wobei das Kühlfluid vom Austrittsende der Kapillare direkt auf den Hautdefekt aufgesprüht wird, so dass ein dünner Film auf dem Hautdefekt gebildet wird, und wobei der dünne Film dann verdampfen gelassen wird und so die Zellen des Hautdefekts unterkühlt werden.

16. Verfahren gemäss Anspruch 15, wobei eine Vorrichtung verwendet wird, die einen Applikator umfasst, in welchem das Austrittsende der Kapillare angeordnet ist, und wobei der Applikator so auf die Haut aufgesetzt wird, dass er den Hautdefekt umschliesst, und wobei das Kühlfluid dann vom Austrittsende der Kapillare direkt auf den Hautdefekt gesprüht wird.

17. Verfahren gemäss Anspruch 16, wobei eine Vorrichtung verwendet wird, die einen Applikator mit einem ringförmigen Schild umfasst, um zu verhindern, dass Gebiete in der Umgebung des zu behandelnden Hautdefekts von dem Kühfluid beaufschlagt werden, das auf den Hautdefekt gesprüht wird.

## Revendications

1. Dispositif de pulvérisation comportant un réservoir (1) dans lequel est stocké un fluide réfrigérant en surpression sous forme liquide, équipé d'un clapet de sortie de fluide (2), et d'une tête de pulvérisation (3 ; 3b) montée sur le réservoir (1) et possédant un tube capillaire (30 ; 30a; 30b) disposé dans un poussoir (31 ; 31b) de sorte que l'extrémité d'admission (300) du tube capillaire (30 ; 30a ; 30b) soit connectée au clapet de sortie de fluide (2) du réservoir et provoque le passage du fluide de refroidissement dans le tube capillaire (30 ; 30a; 30b) lorsque le clapet de sortie de fluide (2) est actionné, dans lequel le réservoir (1) contient un autre tube capillaire (10) dont l'extrémité d'admission se prolonge dans le fluide de refroidissement stocké sous forme liquide dans le réservoir (1), et dont l'extrémité de sortie est reliée au clapet de sortie de fluide (2), **caractérisé en ce que** le diamètre interne du tube capillaire (30 ; 30a; 30b) et de l'autre tube capillaire (10) se situe entre 0.35 mm et 1 mm, et **en ce que** le fluide de refroidissement est produit sous forme gazeuse sur l'extrémité de sortie du tube capillaire (30 ; 30a; 30b).

2. Dispositif de pulvérisation selon la revendication 1, dans lequel la tête de pulvérisation (3 ; 3b) est munie d'une ouverture réceptrice (33 ; 361b) destinée à recevoir un applicateur (5, 6 ; 5a ; 6b) dans lequel le fluide est guidé vers une cible souhaitée.

3. Dispositif de pulvérisation selon la revendication 2, dans lequel l'applicateur possède une section de tube (5 ; 5a) qui est solidement reliée à la tête de pulvérisation (3).

4. Dispositif de pulvérisation selon la revendication 3, dans lequel la section de tube (5 ; 5a) est reliée à la tête de pulvérisation (3) au moyen d'une liaison par emboîtement-pression.

5. Dispositif de pulvérisation selon l'une quelconque des revendications 3 ou 4, dans lequel la section de tube (5a) possède, à son extrémité libre, une région d'extrémité en forme de cloche (51a) dont le diamètre interne est supérieur au diamètre interne du reste de la section de tube (5a), et dans lequel le tube capillaire (30a) de la tête de pulvérisation (3) est guidé pour traverser la section de tube (5a), et son extrémité de sortie (301a) est disposée dans la région d'extrémité en forme de cloche (51a) de la section de tube (5a).

6. Dispositif de pulvérisation selon l'une quelconque des revendications 3 ou 4, dans lequel l'applicateur possède un corps poreux ou cellulaire (6), placé sur la section de tube (5) à l'extérieur de la tête de pulvérisation (3), et dans lequel la section de tube (5), dans la région (51) dans laquelle le corps poreux ou cellulaire (6) est placé sur la section de tube (5), est munie d'ouvertures traversantes (52) au travers desquelles le fluide de refroidissement peut passer dans le corps poreux ou cellulaire (6).

7. Dispositif de pulvérisation selon l'une quelconque des revendications 1 à 5, dans lequel est placé un capuchon protecteur susceptible de pivoter (4), permettant un mouvement de pivotement entre une position ouverte et une position fermée, et susceptible d'être fixé (35, 400) dans la position ouverte.

8. Dispositif de pulvérisation selon la revendication 6 et la revendication 7, dans lequel le capuchon protecteur susceptible de pivoter (4) est conçu de telle sorte que, dans l'état fermé du capuchon protecteur (4), le clapet de sortie de fluide (2) peut être actionné afin que le fluide de refroidissement puisse passer dans le corps poreux ou cellulaire (6) rendu inaccessible par le capuchon protecteur (4).

9. Dispositif de pulvérisation selon la revendication 2, dans lequel le dispositif de pulvérisation comporte un adaptateur (5b), disposé sur l'extrémité de sortie du tube capillaire (30b), l'adaptateur (5b) étant conçu pour être relié à l'applicateur (6b) qui guide le fluide de refroidissement vers la cible souhaitée.

10. Dispositif de pulvérisation selon la revendication 9, dans lequel l'adaptateur (5b) possède une forme s'approchant essentiellement de celle d'un tuyau, l'adaptateur (5b) étant conçu pour se déplacer sur le tube capillaire (30b) et pour être relié à une touche tactile (32b) sur laquelle est monté le poussoir (31b) et dans lequel est disposé le tube capillaire (30b).

11. Dispositif de pulvérisation selon la revendication 10, dans lequel l'adaptateur (5b) est relié à la touche tactile (32b) au moyen d'une liaison par emboîtement-pression (50b, 330b).

12. Dispositif de pulvérisation selon la revendication 10 ou la revendication 11, dans lequel la tête de pulvérisation (3b) comporte un boîtier (36b) possédant un élément de sécurité élastique (34b) évitant normalement toute action sur la touche tactile (32b), et dans lequel la tête de pulvérisation (3b) comporte en outre un patin (35b) disposé de telle sorte qu'il soit mobile pour déformer de manière élastique l'élément de sécurité (34b) afin de permettre une action sur la touche tactile (32b).

13. Dispositif de pulvérisation selon la revendication 12, dans lequel l'applicateur (6b) est conçu et disposé pour être appuyé contre le patin (35b) afin d'assurer le mouvement du patin (35b) et de déformer de manière élastique l'élément de sécurité élastique (34b) et permettre une action sur la touche tactile (32b).

14. Dispositif de pulvérisation selon l'une quelconque des revendications 9 à 13, dans lequel l'applicateur (6b) comporte une partie de forme essentiellement cylindrique (63b) possédant des fentes (630b), et dans lequel l'applicateur (6b) comporte en outre une protection annulaire (61b) autour de son ouverture de sortie (62b) afin d'éviter que les régions situées autour de la cible ne subissent un impact du fluide de refroidissement.

15. Procédé de traitement de défauts de la peau, dans lequel un fluide de refroidissement est appliqué au défaut de la peau à traiter pendant une période suffisante pour la destruction des cellules du défaut de la peau, dans lequel le fluide de refroidissement est pulvérisé sur le défaut de la peau en utilisant un dispositif d'apport du fluide de refroidissement sous forme gazeuse sur une extrémité de sortie d'un tube capillaire, et dans lequel le fluide de refroidissement est pulvérisé à partir de l'extrémité de sortie du tube capillaire, directement sur le défaut de la peau, afin de former un film mince sur le défaut de la peau, et dans lequel le film mince peut ensuite s'évaporer, ce qui assure une super-réfrigération des cellules du défaut de la peau.

16. Procédé selon la revendication 15, dans lequel un dispositif utilisé possède un applicateur dans lequel est disposée l'extrémité de sortie du tube capillaire, et dans lequel l'applicateur est placé sur la peau de manière à circonscrire le défaut de la peau, et dans lequel le fluide de refroidissement est ensuite pulvérisé depuis l'extrémité de sortie du tube capillaire directement sur le défaut de la peau.

17. Procédé selon la revendication 16, dans lequel un dispositif utilisé possède un applicateur, muni d'une protection annulaire, afin d'éviter que les régions situées autour du défaut de la peau à traiter ne subissent l'impact du fluide de refroidissement pulvérisé sur le défaut de la peau.
